# EUROPEAN PATENT SPECIFICATION

(11) **EP 4 532 000 B1**
(45) Date of publication and mention of the grant of the patent: **08.04.2026**
(21) Application number: 23736560.6
(22) Date of filing: 30.05.2023
(51) Int. Cl.: A61N 1/372, A61M 25/00, A61N 1/375

(54) **DELIVERY SYSTEM FOR OVER THE WIRE IMPLANTATION OF A MEDICAL DEVICE**
ABGABESYSTEM FÜR DIE ÜBERDRAHTIMPLANTATION EINER MEDIZINISCHEN VORRICHTUNG
SYSTÈME DE POSE POUR L'IMPLANTATION SUR FIL D'UN DISPOSITIF MÉDICAL

(30) Priority: 01.06.2022 US 202263365671 P
(43) Date of publication of application: 09.04.2025
(73) Proprietor: Medtronic, Inc., Minneapolis, Minnesota 55432 (US)
(72) Inventor: DRAKE, Ronald A., Mounds View, Minnesota 55112 (US); STENER, Lester O., Mounds View, Minnesota 55112 (US); BONNER, Matthew D., Mounds View, Minnesota 55112 (US)
(74) Representative: Zimmermann & Partner Patentanwälte mbB
(86) International application number: PCT/US2023/067630
(87) International publication number: WO 2023/235707

(56) References cited:
- US-A1- 2011 251 662
- US-A1- 2011 270 340
- US-A1- 2015 273 207
- US-A1- 2021 121 689

## Description

### BACKGROUND

The leadless pacemaker, which is significantly smaller than a conventional pacemaker coupled to one or more transvenous leads, is a self-contained generator and electrode system configured to be implanted directly into the heart. The leadless pacemaker, which does not utilize leads extending from out of the heart of a patient, eliminates several complications associated with transvenous pacemakers and leads such as, for example, pocket infections, hematoma, lead dislodgment, and lead fracture. The leadless pacemaker also has cosmetic appeal because there is no chest incision or visible pacemaker pocket.

The leadless pacemaker includes one or more electrodes on its outer housing to deliver therapeutic electrical signals and/or sense intrinsic depolarizations of the heart. Intracardiac medical devices may provide cardiac therapy functionality, such as sensing and pacing, and may also be used to treat either atrial or ventricular arrhythmias or fibrillation.

The leadless pacemaker device may be implanted via a femoral vein (or jugular vein) transcatheter approach, and requires no chest incision or subcutaneous generator pocket. The system utilized to deploy the leadless pacemaker includes a catheter having a distal end with a delivery cup housing the self-contained generator and electrode system. The delivery cup is maneuvered into the proper position, e.g., in the right atrium at or near the triangle of Koch (TOK) or the right ventricle, under fluoroscopic visualization or using a sonogram produced by an ultrasound imaging system.

Interventional guidewires, known to those skilled in the art of minimally invasive medical procedures, are useful for providing access to remote sites within a patient's body, for example, to deliver diagnostic and/or therapy devices to the sites. In many instances, a guidewire is inserted into the body and steered into position at a target site so that a catheter can be guided to the site over the guidewire. The catheter, as part of an interventional medical system, may be configured to deliver a medical device to the site.

In some over-the-wire procedures, an elongate tapered dilator/introducer system may be used to initiate vascular access and navigate patient vasculature. Using such a system, a guidewire is inserted in the vessels of the patient and guided to the right atrium of the heart. A tapered dilator is inserted into an elongate tubular introducer, with the tapered tip of the dilator extending beyond the tip of the introducer. The guidewire, which is already in the body of the patient, is inserted through the dilator, and the dilator/introducer are navigated over the guidewire into the body. Once the tip of the introducer is positioned in the heart, the guidewire and the dilator are removed, leaving the tubular introducer in place. A deployment catheter including a distal delivery cup housing the leadless pacemaker is then moved through the introducer into the heart, and the leadless pacemaker is anchored at a target implantation site.
US 2011/270340 A1 relates to two-stage delivery systems and methods for fixing a leadless implant to tissue.

### SUMMARY

In some patients, vascular access can present a clinical challenge in the implantation of a medical device. In some cases, the outer diameter of the dilator/introducer system used to implant a medical device such as a leadless pacemaker can be up to about 32 French (Fr) (10.7 mm). If a patent has distorted or tortuous vessels, or even smaller vessels, guiding the dilator/introducer system to an implantation site to deploy the medical device can be difficult, even with the assistance of a guidewire. The large outside diameter of the dilator/introducer system may increase the difficulty of navigating the system through the vessels of a patient and may increase the possibility of vascular damage. For at least these reasons, practitioners have not often used over-the-wire guidance to deploy larger medical devices such as leadless pacemakers.

In general, the present disclosure is directed to a shuttle apparatus that replaces, and eliminates the need for, the dilator/introducer system that practitioners currently rely on to navigate through patient vasculature and into the heart for implantation of a medical device. The shuttle apparatus of the present disclosure releasably engages a container on a delivery catheter configured to deploy a medical device such as, for example, a leadless pacemaker. The shuttle apparatus includes a distal end including a tapered semiconical introducer member that may be easily maneuvered through the vessels of a patient during an implantation procedure. The shuttle apparatus further includes a proximal end with a hollow frustoconical plug member shaped to fit into a bore in the container, and this plug member is also shaped such that the shuttle apparatus can be easily retracted from the vessels of the patient following implantation of the medical device. The shuttle apparatus further includes a collar member with a distal portion on the introducer member and a proximal portion on the plug member. The collar member engages an elongate tubular guidewire conduit so that a practitioner can utilize a system including the shuttle apparatus and medical device delivery catheter to implant the medical device using an over-the-wire implantation procedure.

Since the shuttle apparatus eliminates the need for the dilator/introducer, and fits within the bore in the container on the delivery catheter, the diameter of a system including the shuttle apparatus and the medical device delivery catheter has a reduced outside diameter. For example, in some implantation systems configured to deploy a medical device such as a leadless pacemaker, the overall outside diameter of a system including the shuttle apparatus and a delivery cup housing the leadless pacemaker is no more than about 30 Fr (10 mm), a reduction of about 6% compared to a conventional dilator/introducer system. In addition, since the dilator/introducer is not needed, the French size of the outside diameter of the system proximal the delivery cup is even smaller, which can ease navigation of the system through the vasculature of the patient. For example, in many cases the outside diameter of the system including shuttle apparatus and delivery catheter does not exceed the outside diameter of the delivery cup or the delivery catheter themselves, which can be as small as 18 Fr (6 mm). In addition, the shuttle apparatus is more flexible than the tubular introducer in a region distal the delivery cup, which can further simplify navigation and placement of the delivery cup.

The semiconical introducer member includes a tapered distal tip so that the shuttle apparatus can be navigated through patient vasculature to a preferred implant site for the medical device. Once the container or delivery cup for the medical device has reached the preferred implant site, the shuttle apparatus may be ejected from the delivery cup, and in some examples can provide a radiopaque marker to assist a practitioner in finding a preferred implant site in the heart. Following implantation of the medical device, the frustoconical plug member includes a tapered surface so that the shuttle apparatus may be quickly and easily extracted from patient vasculature. The collar member on the shuttle apparatus provides secure engagement with a guidewire for both guidance to the implant site and removal following implant, which makes the shuttle apparatus of the present disclosure highly useful for over-the-wire implantation procedures.

In addition, in some examples the shuttle apparatus may be produced by a process such as, for example, injection molding, using readily available and inexpensive polymeric materials.

In some examples, the implantation system of the present disclosure further includes an optional introducer sleeve that can be slidably moved over an external surface of the delivery catheter including the distal container or delivery cup. The introducer sleeve can increase the column strength of the catheter as the implantation system is navigated through patient vasculature, and can be moved along the catheter to a desired location to change a radius of curvature of the delivery catheter and improve the positioning of the container or delivery cup.

In one aspect, the present disclosure is directed to a shuttle apparatus configured to detachably engage a delivery system for an implantable medical device. The shuttle apparatus includes a distal portion having a generally semiconical introducer member with a proximal base and a distal apex; a proximal portion including a hollow, generally frustoconical plug member with a base adjacent to the base of the introducer member; and a collar member with a distal end on the introducer member and a proximal end on the plug member, wherein the proximal end of the collar member includes a cantilevered arm.

In another aspect, the present disclosure is directed to a system for delivery of a leadless pacemaker. The system includes a catheter having an elongate flexible tubular body with a proximal end and a distal end, wherein the distal end of the tubular body includes a container configured to releasably retain therein the leadless pacemaker, and wherein the container has a longitudinal bore extending from a proximal end to a distal end thereof; a shuttle apparatus configured for releasable insertion into the container of the catheter, wherein the shuttle apparatus includes a distal end with a tapered introducer member, a proximal end with a plug member adjacent to the introducer member and an external surface configured to fit within the bore in the container; and a collar member with a cylindrical bore configured to engage a guidewire or guidewire conduit, the collar member having a distal end that extends along an altitude of the introducer member, and a proximal end that extends over the plug member, wherein the proximal end of the collar member includes a cantilevered arm deflectably moveable into and out of engagement with the container.

In another aspect, the present disclosure is directed to a method for implanting a leadless pacemaker. The method includes inserting a distal end of a guidewire into vasculature of a patient; inserting a proximal end of the guidewire into a collar member on a shuttle apparatus and into a guidewire conduit attached to the collar member, wherein the collar member has a cantilevered arm clampable against an external surface of a container housing the leadless pacemaker, the shuttle apparatus further including: a distal end with a generally semiconical introducer member, and a proximal end with a generally frustoconical plug member, wherein the plug member has an external surface configured to fit in a bore of the container; translating the container over the guide wire and through the vasculature of the patient to an implant site; ejecting the shuttle assembly from the container; deploying the leadless pacemaker from the container; implanting the leadless pacemaker at the implant site; removing the catheter from the vasculature of the patient; and withdrawing the guidewire conduit and the shuttle apparatus attached thereto from the vasculature of the patient.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a schematic illustration of a conventional system for guiding and implanting a leadless pacemaker into a target tissue.
FIG. 2A is an overhead view of an embodiment of a shuttle apparatus of the present disclosure.
FIG. 2B is a side view of an embodiment of a shuttle apparatus of the present disclosure.
FIG. 2C is a bottom view of an embodiment of a shuttle apparatus of the present disclosure.
FIG. 2D is an overhead perspective view of an embodiment of a shuttle apparatus of the present disclosure.
FIG. 2E is a bottom perspective view of an embodiment of a shuttle apparatus of the present disclosure.
FIG. 2F is a front end view of an embodiment of a shuttle apparatus of the present disclosure.
FIG. 2G is a rear end view of an embodiment of a shuttle apparatus of the present disclosure.
FIG. 3A is an exploded perspective view of an embodiment of a medical device implantation system including the shuttle apparatus of the present disclosure.
FIG. 3B is a cross-sectional view of the medical device implantation system of FIG. 3A.
FIG. 3C is a detail of the cross-sectional view of the medical device implantation system of FIG. 3B.
FIG. 3D is a side view of the medical device implantation system of FIG. 3A with the shuttle apparatus fully inserted in the delivery cup on the catheter.
FIGS. 4A-4B illustrate details of the cross-sectional view of the medical device implantation system of FIGS. 3A-3D showing the ejection of the medical device from the delivery cup and the disengagement of the shuttle apparatus of the present disclosure from the delivery cup.
FIG. 5A is a perspective view of an embodiment of a medical device implantation system showing a shuttle apparatus having a cantilevered arm without a snap-fit connector for engagement with the delivery cup housing a medical device.
FIG. 5B is a perspective view of the medical device implantation system of FIG. 5A showing the cantilevered arm engaging an external surface of the delivery cup.
FIG. 5C is a perspective view of the medical device implantation system of FIG. 5A showing the shuttle apparatus fully engaged with the delivery cup.
FIG. 6 is a flow chart illustrating an embodiment of a method for implanting a medical device using the shuttle apparatus and implantation system of the present disclosure.
FIGS. 7A-7B are illustrations of a medical device implantation system including a catheter having thereon a slidable introducer sleeve.

Like symbols in the drawings indicate like elements.

### DETAILED DESCRIPTION

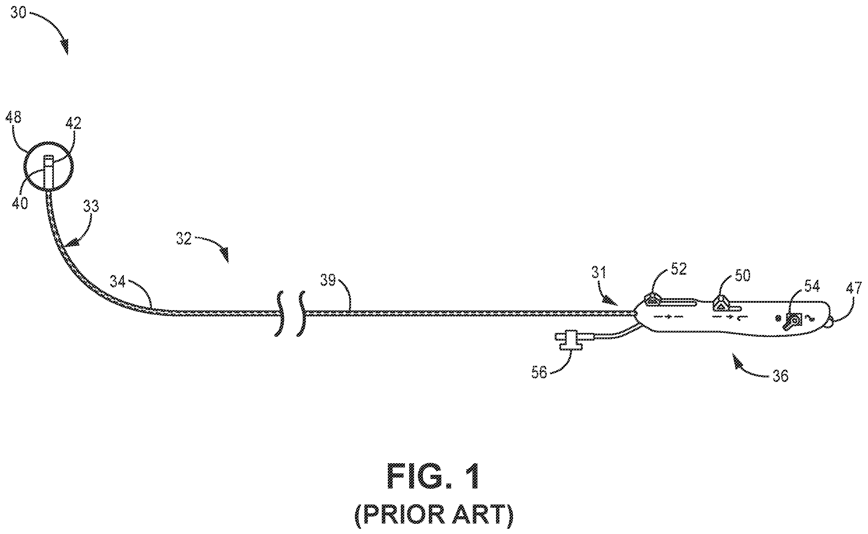

FIG. 1 is a schematic illustration (which is not to scale) of a conventional system 30 for guiding a medical device through vasculature of a patient to a target tissue location for implantation. While the present disclosure exemplifies systems and methods for implantation of leadless pacemakers, it should be understood that the systems of the present disclosure can be utilized to implant any type of medical device such as, for example, temporary or permanent pacemakers with leads, cardiac monitors, pressure sensors, cardiac defibrillators, stents including coronary stents, joint implants, interocular lenses, neurostimulators, pumps, and the like.

In the example of FIG. 1, the system 30 is configured to deliver a leadless pacemaker to a desired implant location so that the leadless pacemaker may be implanted into a target cardiac tissue. The system 30 includes an elongate tubular catheter 32 having a body 34 with a bore 39 that extends from a proximal end 31 to a distal end 33 thereof. In some examples, which are not intended to be limiting, the catheter body 34 has a length of about 75 centimeters (cm) to about 150 cm.

The proximal end 31 of the catheter body 34 is connected to a control handle 36 that can be used to deflect the catheter body 34 and deploy a pacing capsule 40. The pacing capsule 40 is releasably retained at the distal end 33 of the catheter body 34 in a delivery cup or container 42. Suitable leadless pacemaker pacing capsules 40 include, but are not limited to, those available from Medtronic, Inc., Minneapolis, MN, under the trade designation MICRA. The pacing capsule 40 includes a self-contained pulse generator, electronics for control of the pacemaker and electrode system that is implantable into cardiac tissue, and do not require leads or a subcutaneous pacemaker pocket like a transvenous pacemaker system.

The catheter body 34 may be placed in a suitable vein of a patient such as, for example, the femoral vein, and moved through the venous system to place a distal end 48 of the delivery cup 42 at a predetermined location in the heart. In some examples, the catheter body 34 may be deflected using an optional curve deflection control 50 on the handle 36. In some examples, the location of the catheter body 34 and a distal region 48 of the delivery cup 42 is monitored with a fluoroscopic and/or ultrasonic imaging system, and an X-ray or sonogram image of the catheter body 34 and the delivery cup 42 is used to precisely position the delivery cup 42 in the heart. In some embodiments, during placement procedures a proximal portion of the pacing capsule 40 can optionally be tethered via a mechanical tether (not shown in FIG. 1) such as, for example, a tether pin 47.

Once the delivery cup 42 is positioned at within a selected region of the heart such as, for example, the Triangle of Koch (TOK) region or the coronary sinus, the pacing capsule 40 is deployed from the delivery cup 42 via manipulation of the pacing capsule 40 or engagement of an optional deployment control 52 on the handle 36. The pacing capsule 40 can be implanted into the cardiac tissue using, for example, an arrangement of spring-loaded or self-expanding metal tines, a screw-in metal helix, and combinations thereof. After the pacing capsule 40 is implanted in the target tissue of the heart, a tether lock 54 on the handle 36 is released, and the catheter body 34 is withdrawn from the vascular system of the patient. The pacing capsule remains in position within the heart to provide suitable pacing therapy for the patient.

In some examples, the handle 36 may optionally include a fluid port 56, which can provide a fluid flush through the bore 39 of the catheter body 34 using a fluid such as, for example, water, saline, and the like.

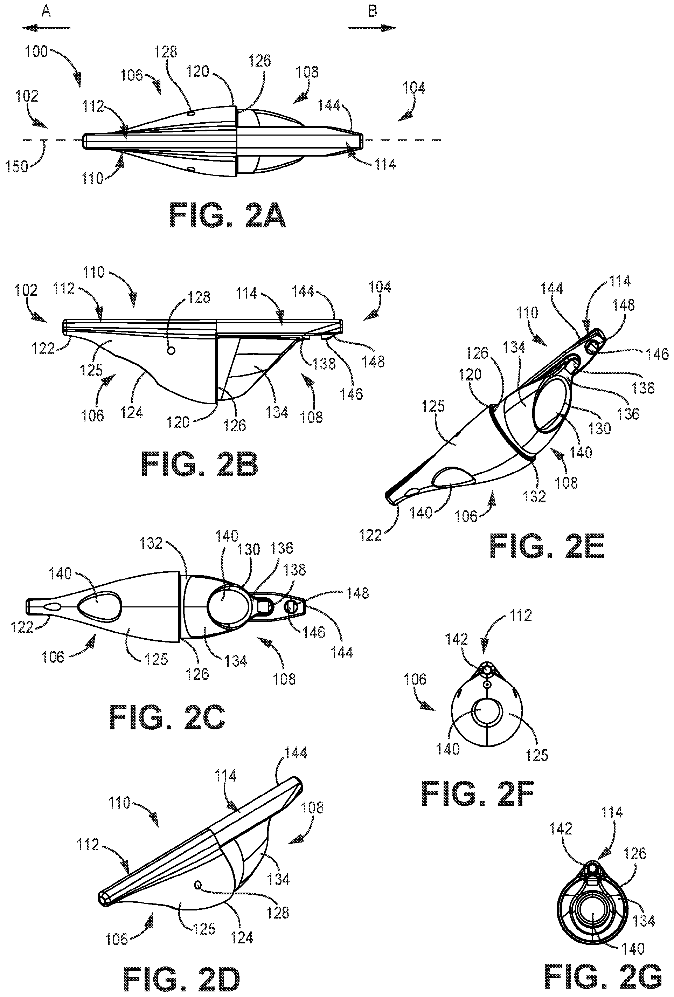

FIGS. 2A-G show a shuttle apparatus 100 that includes a distal portion 102 and a proximal portion 104. The distal portion 102 includes a generally semiconical introducer member 106, and the proximal portion 104 includes a generally frustoconical plug member 108. A collar member 110 includes a distal portion 112 attached to the introducer member 106, and a proximal portion 114 attached to the plug member 108.

The semiconical introducer member 106, which has the shape of a portion of a cone, has a diameter that tapers from a maximum at a proximal semi-circular base 120 to a minimum at a distal tip 122. The introducer member 106 includes a curved external surface 125, and a wall 124 of the introducer member has a non-linear arcuate shape. The narrow diameter of the distal tip 122 and the shape of the arcuate wall 124 make the introducer member 106 atraumatic to patient vasculature as the shuttle apparatus 100 is pushed along a distal direction (arrow A in FIG. 2A) in an implantation procedure. The introducer member 106 further includes an annular shelf portion 126 that extends in a direction normal to a longitudinal axis of the shuttle apparatus 100.

In some examples, the arcuate wall 124 can include an optional port 128. In some examples, the port 128, which extends along a direction normal to the longitudinal axis 150 of the shuttle apparatus 100, may be filled with a radiopaque material such as a metal or metal alloy, to provide fluoroscopic location information about the position of the shuttle apparatus 100 within the vessels or heart of the patient.

The frustoconical plug member 108 has a diameter that tapers from a maximum at a base 132 to a minimum at an annular apex portion 130. The annular apex portion 130 is oriented at an acute angle with respect to a base 132 of the plug member, and at an obtuse angle with respect to the longitudinal axis 150 of the shuttle apparatus 100.

The annular apex portion 130 bounds a bore 140, which extends through the plug member 108 and the introducer member 106. In some examples, the bore 140 may be used to flush the shuttle apparatus 100 during an implantation procedure with a fluid such as water, saline, and the like.

The plug member 108 further includes a wall 134 extending from the base 132 to the annular apex portion 130. The wall 134 is sized and configured to fit within an internal surface of a bore of a delivery container or cup on a delivery catheter for implantation of a medical device (shown in more detail below). The wall 134 has a smooth, rounded shape such that the shuttle device 100 may be pulled in a proximal direction (arrow B in FIG. 2A) without causing undue trauma to the vasculature of a patient. In some examples, the wall 134 may optionally have a shape, be made of selected materials, or have a surface texture, selected to provide a friction fit with the internal bore of the delivery cup for the medical device.

The wall 134 of the plug portion 108 further includes an arcuate ramp 136 that extends away from the annular apex portion 130 and extends to a shoulder 138. The shoulder 138, which is shown in more detail below, extends in a direction generally normal to the longitudinal direction 150 of the shuttle apparatus 100.

The collar member 110 of the shuttle apparatus 100 contacts and extends over both the introducer member 106 and the plug member 108, and includes a generally tubular bore 142 traversing the full length of the collar member 110. The tubular bore 142 is configured to retain an elongate tubular conduit that retains a guidewire (not shown in FIGS. 2A-G, shown in more detail below). The guidewire is confined within the tubular conduit, and moves slidably therewithin during implantation procedures such that the shuttle apparatus 100 can move freely along the length of the guidewire. The distal portion 112 of the collar member 110 extends generally along a height or altitude of the semiconical introducer portion 106, and terminates at the distal tip 122 of the introducer member 106. The proximal portion 114 of the collar member 110 extends beyond the wall 134 of the plug member 108 and forms a cantilevered arm 144.

As explained in more detail below, the cantilevered arm 144 is formed of a material that may be displaced up and down along a direction generally normal to the longitudinal axis 150 of the shuttle apparatus 100 to apply or release a retaining force on a delivery cup retaining an implantable medical device. In the embodiment of FIGS. 2A-G, as discussed in more detail below, the cantilevered arm includes a tab 146 that extends along a direction generally normal to the longitudinal axis 150 of the shuttle apparatus, and forms a component of a snap-fit connector with a corresponding receptacle in a wall of a delivery cup retaining an implantable medical device. In some examples, the tab 146 includes an angled engagement surface 148 to facilitate engagement and disengagement from the corresponding receptacle in the delivery cup.

The introducer member 106, the plug member 108, and the collar member 110 of the shuttle apparatus 100 may be made from a wide variety of materials such as, for example, polymers, or metals such a stainless steel, titanium, or mixtures and alloys thereof. In some examples, the introducer member 106, the plug member 108 and the collar member 110 may each be formed from a relatively hard medical grade plastic, such as 72D PEBAX, Delrin or Nylon, for example, by injection molding. The material may include radiopaque additives and/or echogenic additives to allow the shuttle apparatus to be more visible under fluoroscopy and/or ultrasound.

In some examples, each member of the shuttle apparatus may be formed from the same or different materials. For example, the size, thickness, shape, and material composition of the cantilevered arm 144 may be selected to provide a predetermined amount of engagement force on a delivery cup, while the composition of the wall 125 of the introducer portion 106 may be formed from a softer material than the material selected to make the cantilevered arm 144. In another example, the wall 134 of the plug portion 108 may be formed of a material selected to provide a frictional fit of the plug portion 108 and a delivery cup or container into which the plug portion 108 is to be inserted.

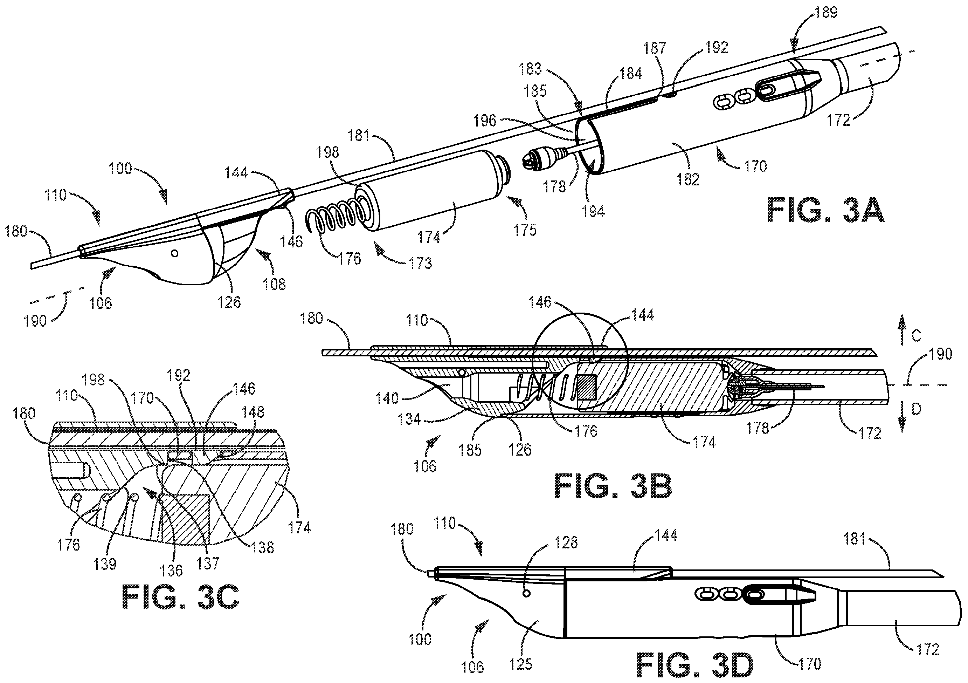

FIGS. 3A-D illustrate a medical device implantation system 200 including the shuttle apparatus 100 of FIGS. 2A-G discussed above. Referring now to the exploded view in FIG. 3A, the system 200 includes the shuttle apparatus 100, which is configured to releasably engage a delivery cup or container 170 on a distal end of a delivery catheter 172. The delivery cup 170 houses an implantable medical device such as, for example, a leadless pacemaker 174. A distal end 173 of the leadless pacemaker 174 includes a helically arranged tine 176 shaped to anchor the pacemaker 174 at a target implantation site in the heart of a patient such as, for example, in a coronary sinus region, a triangle of Koch (TOK) region, and the like. A proximal end 175 of the pacemaker 174 is configured for releasable attachment to a tether 178. A guidewire conduit 181 is retained in the tubular channel 142 of the collar member 110 of the shuttle apparatus 100. A guidewire 180 moves slidably within the conduit 181 such that the shuttle apparatus 100 is retained thereon, but can be moved freely along the length of the guidewire 180.

The delivery cup 170 includes a body 182 with a proximal end 189 connected to the delivery catheter 172, a distal end 183, and a longitudinal bore 194 extending therethrough. The bore 194 includes an internal wall 196.

The distal end 183 of the body 182 includes a linear notch 184 extending generally along a longitudinal axis 190 of the system 200. The notch 184 extends from a distal edge 185 of the delivery cup body 182, and terminates in a U-shaped shaped stop 187. The body 182 of the delivery cup 170 further includes a receptacle 192, which is proximal of the notch 184 and aligned therewith. The receptacle 192, which is shaped to engage with the tab 146 on the cantilevered arm 144 of the shuttle apparatus 100, extends through the body 182 of the delivery cup 170, and forms a component of a snap fit connector with the tab 146.

FIGS. 3B-3D illustrate the system 200 with the leadless pacemaker 174 and the plug member 108 of shuttle apparatus 100 fully inserted into the bore 194 in the delivery cup 170. As shown in FIGS. 3B-3C, when the shuttle apparatus 100 is inserted in the bore 194, the distal edge 185 of the body 182 of the delivery cup 170 abuts the annular ledge portion 126 in the wall 134 of the plug member 108. Helical tine 176 on the leadless pacemaker 174 extends into the bore 140 in the plug member 108.

As the shuttle apparatus 100 is inserted into the bore 194 in the delivery cup 170, the tab 146 is guided along and retained within the linear notch 184. When the tab 146 encounters the U-shaped stop 187, the angular engagement surface 148 on the tab 146 is urged upward, which in turn urges the cantilevered arm 144 in a direction along arrow C normal to the longitudinal axis 190 of the system 200. When the tab 146 lies over the receptacle 192 in the delivery cup 170, the tab 146 and the cantilevered arm 144 move along the direction D, and the tab 146 snaps into engagement and is retained in the receptacle 192. The tab 146 on the cantilevered arm 144 and the receptacle 192 thus form a snap-fit connector that retains the plug member 108 of the shuttle apparatus 100 within the bore 194 of the delivery cup 170. When the tab 146 snaps into place in the receptacle 192, the distal edge 185 of the delivery cup 170 abuts the annular shelf portion 126 on the plug member 108, and the wall 134 of the plug member 108 fully fits within the interior wall 196 of the delivery cup 172.

As shown in detail in FIG. 3C, when the tab 146 is engaged in the receptacle 192, a distal shoulder 198 of the leadless pacemaker 174 is positioned adjacent to the shoulder 138 on the wall 134 of the plug member. As discussed in more detail below, when the leadless pacemaker 174 is advanced distally and moved out of the delivery cup 174, the distal shoulder 198 engages the shoulder 138 on the plug member 108, which has the effect of urging the cantilevered arm 144 away from the delivery cup 170. As the leadless pacemaker 174 is moved in a more distal direction and pushes up the shoulder 138, the shoulder 198 further engages the arcuate ramp 136, which in the example of FIG. 3B-3C includes a linear portion 137 generally parallel to the longitudinal axis 190, and an arcuate section 139 connected to the linear portion 137.

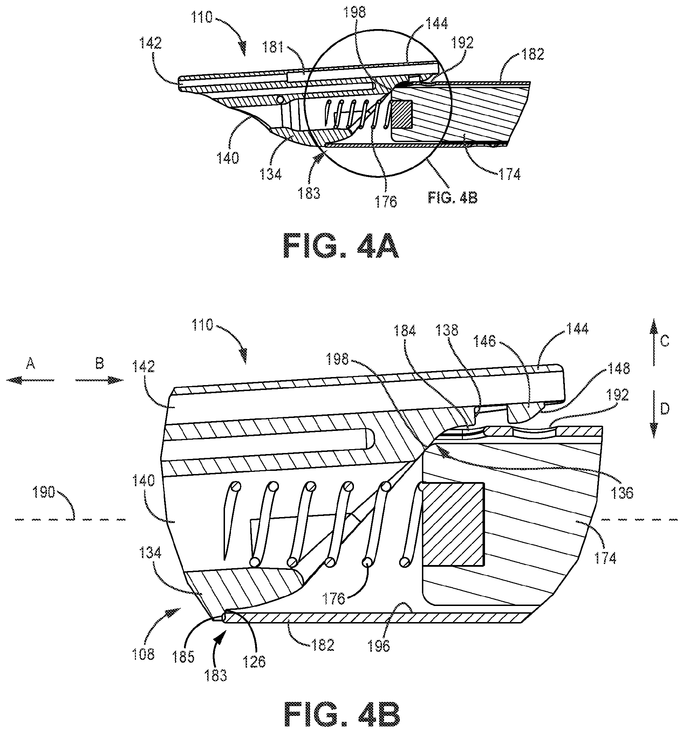

Referring now to FIGS. 4A-4B, as the leadless pacemaker 174 is pushed along a distal direction A, the leadless pacemaker 174 emerges from the distal end 183 of the delivery cup 170. The distal shoulder 198 of the pacemaker 174 is urged against the shoulder 138 on the wall 134 of the plug member 108, which deflects the cantilevered arm 144 upward along the direction of arrow C. As the cantilevered arm is deflected upward, the tab 146 thereon also moves upward and begins to disengage from the receptacle 192 in the wall 182 of the delivery cup 170. As the leadless pacemaker 174 is pushed further in the distal direction A, the distal shoulder 198 thereof engages the arcuate ramp 136 on the wall 134 of the plug member 108, which causes the shuttle apparatus 100 to tilt upward along the direction C and urges the distal edge 185 of the delivery cup 170 against the annular ledge portion 126 on the plug member 108, while further lifting the cantilevered arm 144 and fully disengaging the tab 146 from the receptacle 192 in the delivery cup 170. Further distal movement along the direction of the arrow A of the leadless pacemaker 174 urges the plug member 108 out of the bore 194 in the delivery cup 170 and fully disengages the shuttle apparatus 100 from the delivery cup 170. The disengaged shuttle apparatus 100, which is attached to and suspended on the guidewire conduit 181 (FIG. 4A), falls away from the bore 194 in the delivery cup 170 and exposes tine 176 on the leadless pacemaker 174. Tine 176 may then be employed to implant the leadless pacemaker 174 into a target tissue in the heart, e.g. via further operation of the medical device implantation system 200.

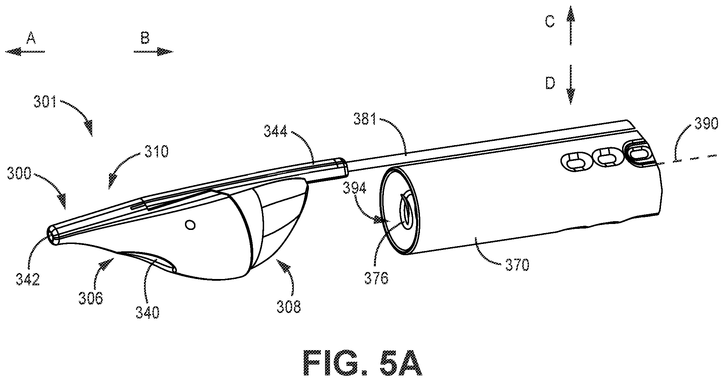

Referring now to FIGS. 5A-5C, in another example a system 301 includes a shuttle apparatus 300 with an introducer member 306, a plug member 308, and a collar member 310. The collar member 310 includes a tubular channel 342 configured to retain a guidewire conduit 381. The shuttle apparatus 300 is configured such that the plug member 308 on the shuttle apparatus 300 is releasably retained in a bore 394 in a delivery cup 370 retaining a leadless pacemaker with tissue-piercing tines 376.

In the embodiment of FIGS. 5A-C, the collar member 310 includes a cantilevered arm 344 formed from a resilient material. Unlike the cantilevered arm 144 described in FIGS. 2-4 above, the cantilevered arm 344 in the system 300 is free of snap-fit connector features configured to releasably engage the delivery cup 370.

As shown in FIG. 5A, prior to insertion of the plug member 308 into the delivery cup or container 370, the resilient cantilevered arm 344 is in a relaxed state and deflected downward in a direction along arrow D normal to a longitudinal axis 390 of the system 300.

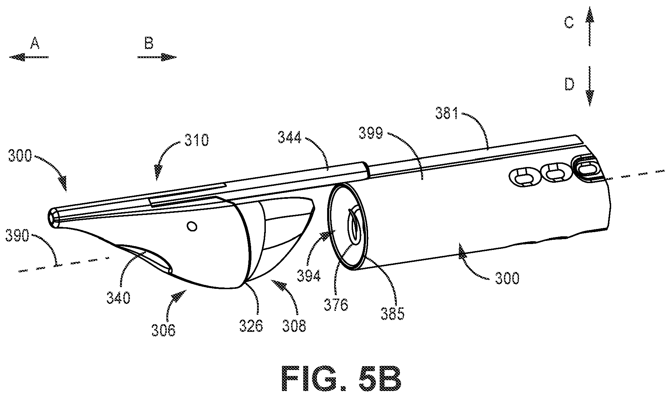

Referring now to FIG. 5B, as the shuttle apparatus 300 is moved proximally in a direction along arrow B, the resilient cantilevered arm 344 engages with an exterior surface 399 of the delivery cup 370. The exterior surface 399 urges the cantilevered arm 344 upward along the direction of arrow C normal to the longitudinal axis 390 of the system 300. The upward movement takes the cantilevered arm 344 out of its original relaxed state, and the deflected arm 344 applies a downward clamping force along the direction of the arrow D against the exterior surface 399 of the delivery cup 370.

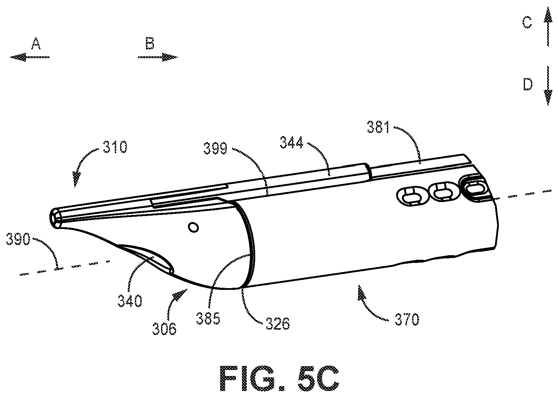

As shown in FIG. 5C, as the shuttle apparatus 300 is moved further in a proximal direction (arrow B), the resilient cantilevered arm 344 extends along the exterior surface 399 of the delivery cup 370, and exerts an increasing clamping force on the surface 399 along a direction of the arrow D. The clamping force between the resilient cantilevered arm 344 and the exterior surface 399 of the delivery cup 370 retains the plug member 308 in the bore 394 of the delivery cup 370, and maintains the position of the shuttle apparatus 300 in the delivery cup 370. The distal edge 385 of the delivery cup 370 abuts the annular ledge portion 326 on the plug portion 308, and the helical fixation tine 376 is housed in the bore 340 in the plug portion 308.

When the leadless pacemaker is pushed distally along the direction of arrow A and is deployed from the delivery cup 370, the clamping force between the resilient cantilevered arm 344 and the surface 399 of the delivery cup 370 is overcome, and the shuttle apparatus 300 is pushed distally along the direction of arrow A and is expelled from the bore 394 of the delivery cup 370, exposing the fixation tine 376. The leadless pacemaker may then be implanted in a target tissue using the exposed fixation tine 376.

After deployment of the leadless pacemaker, shuttle apparatus 300 may be removed from the body. In some examples, shuttle apparatus 300 may include a feature configured to surround the proximal end of the shuttle to allow it to be withdrawn from the vessel without an edge or cavity of shuttle apparatus 300 engaging or snagging any tissue. For example, the feature may include an expandable sleeve that has an inner diameter greater than the outer diameter of the guidewire conduit. This sleeve, which may be formed from a compliant material, may be configured to expand and fold over the proximal end of shuttle apparatus 300 when the sleeve is compressed. In this way, the feature may effectively block any edges or cavities of shuttle apparatus 300 from engaging tissues when shuttle apparatus 300 is withdrawn from the vessel. In one example, the feature may include a soft mesh that expands on compression. In another example, the feature may define multiple slits that enable the feature to collapse longitudinally but expand radially.

In some examples, the shuttle apparatus of the present disclosure can be used to drive a leadless pacing device (such as, for example, those available from Medtronic, Minneapolis, MN, under the trade designation Micra) delivery system over a guidewire in an over-the-wire implantation procedure. The shuttle apparatus of the present disclosure also eliminates the need to utilize a dilator/introducer, which increase the outside diameter of the delivery system for the implantable medical device. The larger diameter delivery system makes navigation of the delivery cup through patient vasculature more difficult, and can increase the likelihood of vascular injury during implantation procedures, while enabling the procedure to be performed with a smaller, faster-healing vascular access opening.

The shuttle apparatus of the present disclosure can also optionally be configured to provide fluoroscopic and/or sonographic location information about the location of the delivery capsule, and even after the shuttle apparatus is ejected from the delivery capsule, the shuttle apparatus remains in the heart to provide location information useful in identifying a preferred implantation site. The attached guidewire also makes the shuttle apparatus easily removable from the vasculature of the patient following implantation of the leadless pacemaker.

In one example, which is not intended to be limiting, the shuttle apparatus of the present disclosure can be inserted in a conventional catheter delivery system such as, for example, the delivery cup for the leadless pacemaker available from Medtronic under the trade designation Micra, straddling the tines of the device.

In one example, a guide wire such as a 0.035" (0.90 mm) guidewire with a ball (or step) on the distal end thereof is inserted into the vasculature (typically the femoral vein). The guidewire is then advanced into a chamber of the heart. The proximal end of the guide wire is then inserted into the collar member on the shuttle apparatus, and into the guidewire conduit attached to the proximal end of the collar member.

The delivery catheter with the attached delivery cup may then be delivered over-the-wire. In some examples, to deflect and position the catheter or the delivery cup in a preferred position to navigate patient vasculature, or to position the delivery cup to provide a preferred orientation for implant, the optional introducer sleeve may be slidably moved along the external surface of the catheter body to a desired position to provide a desired bend angle.

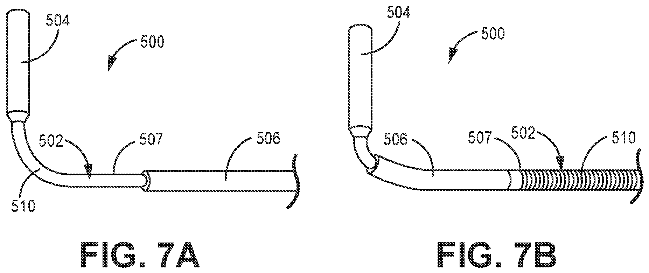

Referring now to FIG. 7A, a medical device implantation delivery system 500 includes a delivery catheter 502 and a delivery cup or container 504 located at a distal end of the catheter and configured to house a medical device such as a leadless pacemaker. A tubular introducer sleeve 506 is placed on an outside surface 507 of a body 510 of the catheter 502, and is slidable along the length of the outside surface 507 of the catheter 502. As shown in FIG. 7A, the introducer sleeve is positioned about relatively far away from the delivery cup 504, and modifies a bending angle of a distal end of the catheter 502. In FIG. 7B, the introducer sleeve 506 is positioned closer to the delivery cup 504, and provides a tighter bend at the distal end of the catheter 502 compared to the bend in FIG. 7A.

The introducer sleeve 506, which is captive on the body 510 of the catheter 502, may be moved by a practitioner to a desired position on the body 510 to provide a selected bending angle for the distal end of the catheter 502, with positions closer to the delivery cup 504 producing tighter bending angles. The introducer sleeve 506 may thus be used to position the delivery cup 504 more precisely for movement through the vasculature of a patient, or to position the delivery cup 504 in a desired position in the heart for implantation of the medical device.

After navigating to near the final position, the shuttle apparatus is no longer needed and is ejected from the delivery cup by partially deploying the leadless pacemaker out of the delivery cup. After being ejected, the shuttle apparatus remains in the chamber of the heart and can provide location information on a fluoroscope and/or ultrasonic imaging system. The leadless pacemaker is then retracted into the delivery cup, and the delivery cup may be moved to a desired position for implant.

The leadless pacemaker may then be deployed from the delivery cup and implanted at the target implant site.

After successful deployment of the leadless pacemaker, the delivery catheter is removed, and the guidewire conduit is then withdrawn. The guidewire conduit, which is attached to the shuttle apparatus, may be withdrawn for the body of the patient to retract the shuttle apparatus out of the body of the patient.

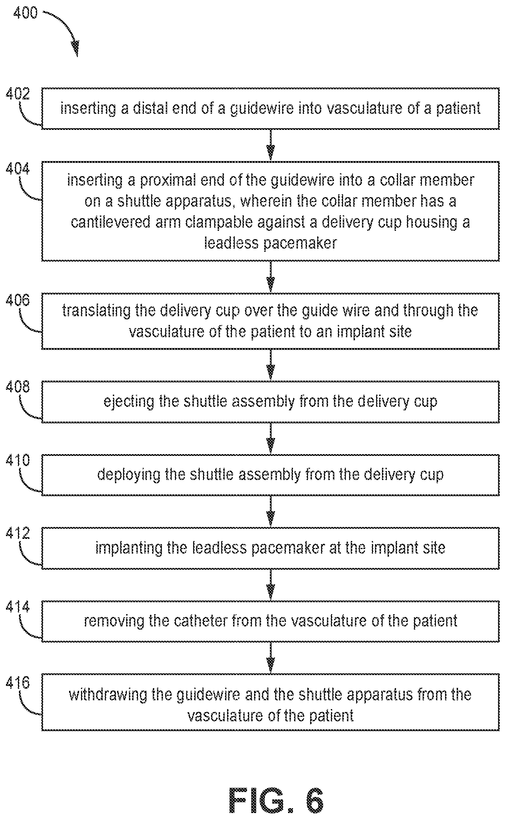

FIG. 6 is a flowchart illustrating method 400 for implanting a leadless pacemaker. The method 400 includes inserting a distal end of a guidewire into vasculature of a patient (402). The method further includes inserting a proximal end of the guidewire into a collar member on a shuttle apparatus (404). The collar member includes a cantilevered arm clampable against an outside surface of a delivery cup housing the leadless pacemaker, and the tine of the leadless pacemaker resides in a bore in the shuttle apparatus.

Step 406 includes translating the catheter carrying the delivery cup over the guide wire and through the vasculature of the patient to an implant site. In some examples, the method includes adjusting the positioning of the catheter or the delivery cup by translating an introducer sleeve over the body of the catheter to modify a bend radius of the catheter or the delivery cup.

Step 408 includes ejecting the shuttle assembly from the delivery cup.

Step 410 includes deploying the leadless pacemaker from the delivery cup.

Step 412 includes implanting the leadless pacemaker at the implant site.

Step 414 includes removing the catheter from the vasculature of the patient.

Step 416 includes withdrawing the guidewire conduit and the shuttle apparatus from the vasculature of the patient.

## Claims

1. A shuttle apparatus configured to detachably engage a delivery system for an implantable medical device, wherein the shuttle apparatus comprises:
a distal portion comprising a generally semiconical introducer member, wherein the introducer member comprises a proximal base and a distal apex;
a proximal portion comprising a hollow, generally frustoconical plug member with a base adjacent to the base of the introducer member; and
a collar member with a distal end on the introducer member and a proximal end on the plug member, wherein the proximal end of the collar member comprises a cantilevered arm.

2. The shuttle apparatus of claim 1, wherein the hollow frustoconical plug member comprises an external surface configured to fit within a bore of a container in the delivery system, wherein the container houses the implantable medical device.

3. The shuttle apparatus of claim 2, wherein the cantilevered arm is deflectably engageable with the body of the container.

4. The shuttle apparatus of claim 3, wherein the cantilevered arm is deflectable in a direction normal to a longitudinal axis of the shuttle apparatus from a relaxed position in engagement with an external surface of the wall of the container to a flexed position out of engagement with the external surface of the wall of the container.

5. The shuttle apparatus of claim 3 or 4, wherein the cantilevered arm and the body of the container comprise a snap fit connector.

6. The shuttle apparatus of claim 5, wherein the snap fit connector comprises a tab on the cantilevered arm, wherein the tab extends in the direction normal to the longitudinal axis of the shuttle apparatus, and a corresponding receptacle in the body of the container configured to engage the tab.

7. The shuttle apparatus of any of claims 1 to 6, wherein the base of the introducer member comprises an annular ledge portion, and wherein the annular ledge portion extends radially outward from the plug member.

8. The shuttle apparatus of any of claims 1 to 7, wherein the plug member comprises an annular apex surface, and wherein the annular apex surface is oriented at an acute angle with respect to the base of the plug member.

9. The shuttle apparatus of claim 8 when dependent on claim 2, wherein the distal end of the container comprises a linear notch extending along a longitudinal axis of the container, and wherein the receptacle is aligned with the notch, and wherein the receptacle is between the notch and a proximal end of the container.

10. The shuttle apparatus of claim 9, wherein the plug member comprises a shoulder at the proximal end thereof, wherein the shoulder extends in a direction normal to a longitudinal axis of the shuttle apparatus, and wherein the shoulder abuts the container at a proximal end of the notch when the tab on the cantilevered arm is in engagement with the receptacle in the body of the container.

11. The shuttle apparatus of claim 10, wherein the shoulder comprises an angled engagement surface.

12. The shuttle apparatus of claim 10, wherein the plug member further comprises a wall with an arcuate ramp, wherein the arcuate ramp is between the shoulder and an annular apex surface on the plug member.

13. The shuttle apparatus of claim 12, wherein the arcuate ramp comprises a first linear portion generally parallel to the longitudinal axis of the shuttle apparatus, and a second angled portion connected to the first linear portion.

14. The shuttle apparatus of any of claims 6 to 13, wherein the tab comprises a proximal end with an angled engagement surface.

15. The shuttle apparatus of any of claims 1 to 14, wherein the shuttle apparatus comprises a bore extending into the plug member, wherein the bore is configured to receive a fixation element of the implantable medical device.

## Patentansprüche

1. Schubschlittenvorrichtung, die zur lösbaren Ineingriffnahme eines Zuführsystems für eine implantierbare medizinische Vorrichtung ausgestaltet ist, wobei die Schubschlittenvorrichtung Folgendes umfasst:
einen distalen Abschnitt, der ein allgemein halbkonisches Einführerglied umfasst, wobei das Einführerglied eine proximale Basis und einen distalen Scheitelpunkt umfasst,
einen proximalen Abschnitt, der ein hohles, allgemein kegelstumpfförmiges Stopfenglied mit einer der Basis des Einführerglieds benachbarten Basis umfasst, und
ein Bundglied mit einem distalen Ende an dem Einführerglied und einem proximalen Ende an dem Stopfenglied, wobei das proximale Ende des Bundglieds einen auskragenden Arm umfasst.

2. Schubschlittenvorrichtung nach Anspruch 1, wobei das hohle kegelstumpfförmige Stopfenglied eine Außenfläche umfasst, die dazu ausgestaltet ist, in eine Bohrung eines Behälters in dem Zuführsystem zu passen, wobei der Behälter die implantierbare medizinische Vorrichtung aufnimmt.

3. Schubschlittenvorrichtung nach Anspruch 2, wobei der auskragende Arm ablenkbar mit dem Körper des Behälters in Eingriff bringbar ist.

4. Schubschlittenvorrichtung nach Anspruch 3, wobei der auskragende Arm in einer orthogonal zu einer Längsachse der Schubschlittenvorrichtung verlaufenden Richtung aus einer entspannten Position in Eingriff mit einer Außenfläche der Wand des Behälters in eine gebogene Position außer Eingriff mit der Außenfläche der Wand des Behälters ablenkbar ist.

5. Schubschlittenvorrichtung nach Anspruch 3 oder 4, wobei der auskragende Arm und der Körper des Behälters einen Schnappverbinder umfassen.

6. Schubschlittenvorrichtung nach Anspruch 5, wobei der Schnappverbinder eine Lasche an dem auskragenden Arm umfasst, wobei sich die Lasche in der orthogonal zu der Längsachse der Schubschlittenvorrichtung verlaufenden Richtung erstreckt, sowie eine entsprechende Aufnahme in dem Körper des Behälters, die zur Ineingriffnahme der Lasche ausgestaltet ist.

7. Schubschlittenvorrichtung nach einem der Ansprüche 1 bis 6, wobei die Basis des Einführerglieds einen ringförmigen Absatzabschnitt umfasst und wobei sich der ringförmige Absatzabschnitt von dem Stopfenglied radial nach außen erstreckt.

8. Schubschlittenvorrichtung nach einem der Ansprüche 1 bis 7, wobei das Stopfenglied eine ringförmige Scheitelfläche umfasst und wobei die ringförmige Scheitelfläche in einem spitzen Winkel in Bezug auf die Basis des Stopfenglieds ausgerichtet ist.

9. Schubschlittenvorrichtung nach Anspruch 8, wenn abhängig von Anspruch 2, wobei das distale Ende des Behälters eine lineare Kerbe umfasst, die sich entlang einer Längsachse des Behälters erstreckt, und wobei die Aufnahme auf die Kerbe ausgerichtet ist und wobei sich die Aufnahme zwischen der Kerbe und einem proximalen Ende des Behälters befindet.

10. Schubschlittenvorrichtung nach Anspruch 9, wobei das Stopfenglied eine Schulter an seinem proximalen Ende umfasst, wobei sich die Schulter in einer orthogonal zu einer Längsachse der Schubschlittenvorrichtung verlaufenden Richtung erstreckt und wobei die Schulter an einem proximalen Ende der Kerbe an dem Behälter anliegt, wenn die Lasche an dem auskragenden Arm mit der Aufnahme in dem Körper des Behälters in Eingriff steht.

11. Schubschlittenvorrichtung nach Anspruch 10, wobei die Schulter eine abgewinkelte Eingriffsfläche umfasst.

12. Schubschlittenvorrichtung nach Anspruch 10, wobei das Stopfenglied ferner eine Wand mit einer bogenförmigen Rampe umfasst, wobei sich die bogenförmige Rampe zwischen der Schulter und einer ringförmigen Scheitelfläche an dem Stopfenglied befindet.

13. Schubschlittenvorrichtung nach Anspruch 12, wobei die bogenförmige Rampe einen ersten linearen Abschnitt, der allgemein parallel zu der Längsachse der Schubschlittenvorrichtung verläuft, und einen zweiten abgewinkelten Abschnitt, der mit dem ersten linearen Abschnitt verbunden ist, umfasst.

14. Schubschlittenvorrichtung nach einem der Ansprüche 6 bis 13, wobei die Lasche ein proximales Ende mit einer abgewinkelten Eingriffsfläche umfasst.

15. Schubschlittenvorrichtung nach einem der Ansprüche 1 bis 14, wobei die Schubschlittenvorrichtung eine Bohrung umfasst, die sich in das Stopfenglied erstreckt, wobei die Bohrung zur Aufnahme eines Fixierungselements der implantierbaren medizinischen Vorrichtung ausgestaltet ist.

## Revendications

1. Appareil de navette configuré pour mettre en prise de manière détachable un système de pose pour un dispositif médical implantable, l'appareil de navette comprenant :
une partie distale comprenant un élément introducteur généralement semi-conique, l'élément introducteur comprenant une base proximale et un sommet distal ;
une partie proximale comprenant un élément de bouchon creux, généralement tronconique, avec une base adjacente à la base de l'élément introducteur ; et
un élément de collier comportant une extrémité distale sur l'élément introducteur et une extrémité proximale sur l'élément de bouchon, l'extrémité proximale de l'élément de collier comprenant un bras en porte-à-faux.

2. Appareil de navette selon la revendication 1, l'élément de bouchon tronconique creux comprenant une surface externe configurée pour s'adapter à l'intérieur d'un alésage d'un récipient dans le système de pose, le récipient logeant le dispositif médical implantable.

3. Appareil de navette selon la revendication 2, le bras en porte-à-faux pouvant venir en prise de manière déviable avec le corps du récipient.

4. Appareil de navette selon la revendication 3, le bras en porte-à-faux pouvant être dévié dans une direction normale à un axe longitudinal de l'appareil de navette d'une position détendue en engagement avec une surface externe de la paroi du récipient à une position fléchie hors engagement avec la surface externe de la paroi du récipient.

5. Appareil de navette selon la revendication 3 ou 4, le bras en porte-à-faux et le corps du récipient comprenant un connecteur à encliquetage.

6. Appareil de navette selon la revendication 5, le connecteur à encliquetage comprenant une patte sur le bras en porte-à-faux, la patte s'étendant dans la direction normale à l'axe longitudinal de l'appareil de navette, et un réceptacle correspondant dans le corps du récipient configuré pour venir en prise avec la patte.

7. Appareil de navette selon l'une quelconque des revendications 1 à 6, la base de l'élément introducteur comprenant une partie de rebord annulaire, et la partie de rebord annulaire s'étendant radialement vers l'extérieur à partir de l'élément de bouchon.

8. Appareil de navette selon l'une quelconque des revendications 1 à 7, l'élément de bouchon comprenant une surface de sommet annulaire, et la surface de sommet annulaire étant orientée selon un angle aigu par rapport à la base de l'élément de bouchon.

9. Appareil de navette selon la revendication 8 lorsqu'elle dépend de la revendication 2, l'extrémité distale du récipient comprenant une encoche linéaire s'étendant le long d'un axe longitudinal du récipient, et le réceptacle étant aligné avec l'encoche, et le réceptacle étant situé entre l'encoche et une extrémité proximale du récipient.

10. Appareil de navette selon la revendication 9, l'élément de bouchon comprenant un épaulement à son extrémité proximale, l'épaulement s'étendant dans une direction normale à un axe longitudinal de l'appareil de navette, et l'épaulement butant contre le récipient à une extrémité proximale de l'encoche lorsque la patte sur le bras en porte-à-faux est en prise avec le réceptacle dans le corps du récipient.

11. Appareil de navette selon la revendication 10, l'épaulement comprenant une surface de mise en prise inclinée.

12. Appareil de navette selon la revendication 10, l'élément de bouchon comprenant en outre une paroi avec une rampe arquée, la rampe arquée étant située entre l'épaulement et une surface de sommet annulaire sur l'élément de bouchon.

13. Appareil de navette selon la revendication 12, la rampe arquée comprenant une première partie linéaire généralement parallèle à l'axe longitudinal de l'appareil de navette, et une seconde partie inclinée reliée à la première partie linéaire.

14. Appareil de navette selon l'une quelconque des revendications 6 à 13, la patte comprenant une extrémité proximale avec une surface de mise en prise inclinée.

15. Appareil de navette selon l'une quelconque des revendications 1 à 14, l'appareil de navette comprenant un alésage s'étendant dans l'élément de bouchon, l'alésage étant configuré pour recevoir un élément de fixation du dispositif médical implantable.
